# EUROPEAN PATENT APPLICATION

(11) **EP 0 791 575 A1**
(43) Date of publication of application: **27.08.1997**
(21) Application number: 97102422.9
(22) Date of filing: 14.02.1997
(51) Int. Cl.: C07C 209/12, C07C 211/63, C07C 215/66, C07D 213/20

(54) **Method of synthesis of a quaternary ammonium salt**

(30) Priority: 16.02.1996 JP 54228/96
(71) Applicant: NIPPON PAINT CO., LTD., Kita-ku, Osaka (JP)
(72) Inventor: Nakano, Shinji, Takatsuki-shi, Osaka-fu (JP)
(74) Representative: Kinzebach, Werner, Dr.

(57) **Abstract**

The present invention provides an improved method of synthesis of a quaternary ammonium salt in which a tertiary amine is reacted with a benzyl halide optionally having on the benzene ring and/or at the α-site one ore more substituent groups inert to the reaction, which method is characterized in that the reaction is carried out in water or in a water-containing organic solvent.

## Description

### FIELD OF THE INVENTION

This invention relates to an improved method of synthesis of a class of quaternary ammonium salts.

### BACKGROUND OF THE INVENTION

Quaternary ammonium halides in which a benzyl group is attached to a nitrogen atom, such as benzalkonium chloride, are widely used as bactericides. Pyridinium or anilinium halides also having a benzyl group are raw materials for preparation of heat latent cationic polymerization initiators (heat latent acid catalysts) disclosed, for example, in the applicant's Japanese Unexamined Patent Publication No. H1-96169.

In general, quaternization reactions employing a benzyl halide as a quaternizing agent are carried out in polar solvents such as alcohols, DMF or acetonitrile. However, as is evident from the examples in the above mentioned patent application in which it takes 3 days in methanol at 40°C, those reactions are not efficient in an organic solvent. Thus there exists a need for a method of synthesis that can provide this type of quaternary ammonium salts, more efficiently , i.e., within a shorter reaction time with higher yields.

### SUMMARY OF THE INVENTION

The inventors discovered that water, if present in the reaction system, could remarkably shorten the reaction time required in the above quaternizing reaction and greatly increase the rate of conversion of the tertiary amine to a corresponding quaternary ammonium salt.

Based upon this finding, the present invention provides a method of synthesis of a quaternary ammonium salt wherein a tertiary amine is reacted with a benzyl halide optionally having on the benzene ring and/or at the α-site one ore more substituent groups inert to the reaction, which method is characterized in that said reaction is carried out in water or in a water-containing organic solvent.

### DETAILED DISCUSSION

The quaternizing agents used in the present invention are benzyl halides or substituted benzyl halides having on the benzene ring and/or at the α-site one or more substituent groups inert to the reaction. Non-limiting examples of such inert substituent groups on the benzene ring include, for example, hydroxyl, alkyl, alkenyl, alkoxy, nitro, cyano or alkoxycarbonyl, and those of inert substituent groups at the α-site include alkyl.

A preferred quaternizing agent has the following formula, 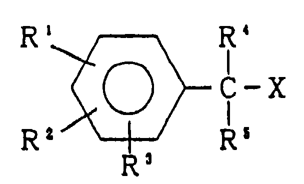 wherein R¹, R² and R³ independently denote hydrogen or one of the above-identified inert groups; R⁴ and R⁵ independently denote hydrogen or alkyl; and X denote halogen, preferably chloro. Specific examples include benzyl chloride, 4-methylbenzyl chloride, 4-t-butylbenzyl chloride, 4-vinylbenzyl chloride, 4-chlorobenzyl chloride, 2,6-dichlorobenzyl chloride, 4-nitrobenzyl chloride, 4-methoxybenzyl chloride, α-methylbenzyl chloride, and corresponding bromides.

Any tertiary amine may be quaternized. However, those amines are preferred which have a pKa of 9.0 or lower in order to allow smoother proceeding of the reaction. Tertiary amines are selected according to the use of the aimed product. For example, when the purpose is the synthesis of a bacteriocidal quaternary ammonium salt such as benzalkonium chloride, such a trialkyl amine is used in which one of the alkyl groups is a long-chain alkyl such as dodecyl, tetradecyl, hexadecyl, or octadecyl, and the remaining groups are either methyl or ethyl.

However, the method of the present invention provides an especially significant advantage in the synthesis of a quaternary ammonium cationic moiety of the heat latent acid catalysts disclosed in the above-cited Japanese Unexamined Patent Publication No. H1-96169. In this case, one of the tertiary amine to be quaternized is a pyridine base having the formula, 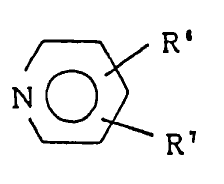 wherein R⁶ and R⁷ independently denote hydrogen or one of the inert substituent groups identified above.
Specifically, they are, for example, pyridine, 4-picoline, 2,3-lutidine and its positional isomers, 2-, 3- or 4-vinylpyridine, 4-cyanopyridine, 2-methoxypyridine and the like.

Another type of tertiary amines is an aniline derivative having the formula, 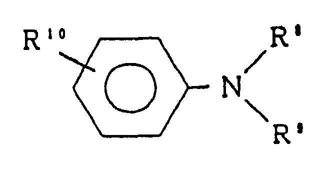 wherein R ⁸ and R⁹ independently denote alkyl or alkenyl; R¹⁰ denotes hydrogen or one of the inert substituent groups identified above. Specifically, they are, for example, N,N-dimethylaniline, N,N-dimethyltoluidine and the like.

The reaction may be carried out between equimolar amounts of a quaternizing agent and a tertiary amine, in water or in a water-containing organic solvent at an temperature from room temperature to the boiling point, preferably at a temperature ranging from 40 to 70 °C. When a water-containing organic solvent is used, the amount of water in the reaction system should be at least equimolar to the amount of the benzyl halide quaternizing agent, more preferably, five times or more of the molar amount thereof.

Examples of solvents that may be used include alcohols such as methanol, ethanol and isopropanol; aromatic hydrocarbons such as benzene and toluene; esters such as ethyl acetate and butyl acetate; ketones such as acetone and methylisobutylketone; chlorinated hydrocarbons such as dichloromethane and dichloroethane; cyclic ethers such as tetrahydrofuran and 1,4-dioxane; amides such as dimethylformamide; nitriles such as acetonitrile; nitro-compounds such as nitrobenzene and nitromethane. Among these, water-miscible organic solvents are preferred, such as methanol, ethanol, isopropanol, tetrahydrofuran, dimethylformamide (DMF) and acetonitrile. The concentration of reactants in the reaction system is preferably not less than 10 % by weight.

After the reaction is completed, the aimed quaternary ammonium salt may be recovered and purified from th reaction mixture according to a conventional method. For example, an addition of a solvent to which the quaternary ammonium salt is only slightly soluble followed by cooling to allow precipitation of crystals, or, simple evaporation in vacuo followed by recrystallization from the residue. However, in the case the produced quaternary ammonium salt is utilized as the cationic component of a heat latent acid catalyst, it is also possible to directly add to the reaction mixture an aqueous solution of, for example, sodium hexafluoroantimonate, and isolate the quaternary ammonium as hexafluoroantimonate salt. Similar procedures are followed in the case that halide anion is exchanged for a different anion such as hexafluoroarsenate, hexafluorophosphate, tetrafluoroborate, triflate, aromatic sulfonate (e.g., benzenesulfonate, toluenesulfonate, docecylbenzenesulfonate).

### EXAMPLE 1

### N-benzyl-N,N-dimethylanilinium chloride

12.65 g (0.1 mole) of benzyl chloride and 12.12 g (0.1 mole) of N,N-dimethylaniline was added with 3.2 g of methanol and stirred to dissolve. To this was added 9.0 g (0.5 mole) of water, and the mixture heated to 70°C. The rate of conversion of benzyl chloride to the quaternary ammonium salt was followed over time by means of ¹H-NMR. The rate of conversion was found to reach 100 % after a reaction time of 3.3 hours.

For comparison, a reaction was carried out under the same conditions except that no water was added. The rate of conversion was 73 % after a reaction time of 5 hours, and there was no increase in the conversion rate thereafter.

To the reaction mixture that had reached the conversion rate of 100 % was added 150 g of acetone, and the mixture was allowed to stand overnight at 5 °C. Precipitated crystals were filtered and dried to give 19.2 g of the titled compound as white cubic crystals. The mother liquor was concentrated, added with about 50 g of acetone and cooled likewise to precipitate crystals, giving 4.9 g of the titled compound. Total 24.1 g, Yield; 98 %. M.p. 184.5-185.2°C

### EXAMPLE 2

### N-(4-methylbenzyl)-N,N-dimethylanilinium chloride

14.6 g (0.2 mole) of 4-methylbenzyl chloride and 24.24 g (0.2 mole) of N,N-dimethylaniline was added with 14.6 g of dimethylformamide and stirred to dissolve. To this was added 18.0 g (1.0 mole) of water, and the mixture heated to 70°C. The rate of conversion reached 100 % after the reaction time of 2 hours. The yield of the aimed crystals was 95 %.

For comparison, a reaction was carried out without addition of water. Conversion rate was not more than 10 % even after the reaction time of 7 hours.

### EXAMPLE 3

### 1-(4-chlorobenzyl)-4-cyanopyridinium chloride

16.05 g (0.1 mole) of 4-chlorobenzyl chloride and 10.4 g (0.1 mole) of 4-cyanopyridine was added with 30 g of tetrahydrofuran and stirred to dissolve. To this was added 18.0 g (1.0 mole) of water and reaction was carried out at 90 °C. The rate of conversion reached 100 % after 5 hours of reaction. The yield of the aimed crystals was 95 %.

### EXAMPLE 3

### N-(4-methylbenzyl)-N,N-dimethylanilinium chloride

14.6 g (0.2 mole) of 4-methylbenzyl chloride and 24.24 g (0.2 mole) of N,N-dimethylaniline was added with 18.0 g (1.0 mole) of water, and the mixture heated to 60°C. The rate of conversion reached 100 % after 4 hours of reaction.

The yield of the aimed crystals was 95 %.

## Claims

1. A method of synthesis of a quaternary ammonium salt wherein a tertiary amine is reacted with a benzyl halide optionally having on the benzene ring and/or at the α-site one ore more substituent groups inert to the reaction, which method is characterized in that said reaction is carried out in water or in a water-containing organic solvent.

2. The method of claim 1, wherein said benzyl halide is a compound of the formula, 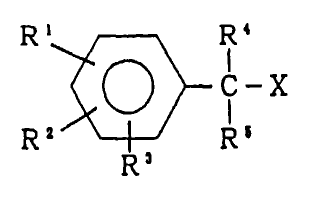 wherein R¹, R² and R³ independently denote hydrogen, hydroxyl, halogen, alkyl, alkenyl, alkoxy, nitro, cyano or alkoxycarbonyl; R⁴ and R⁵ independently denote hydrogen or alkyl; and X denote halogen.

3. The method of claim 1 or 2, wherein said tertiary amine is a compound of the formula, 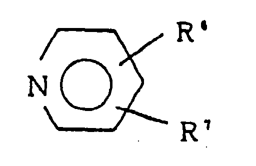 wherein, R⁶ and R⁷ independently denote hydrogen, hydroxyl, halogen, alkyl, alkenyl, alkoxy, nitro, cyano or alkoxycarbonyl.

4. The method of claim 1 or 2, wherein said tertiary amine is a compound of the formula, 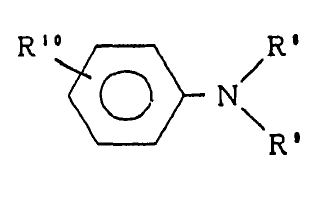 wherein R⁸ and R⁹ independently denote alkyl or alkenyl; R¹⁰ denote hydrogen, hydroxyl, halogen, alkyl, alkenyl, alkoxy, nitro, cyano or alkoxycarbonyl.

5. The method of one of claims 1 to 4, wherein the amount of water in the reaction system is at least equimolar to the amount of said benzyl halide.

6. The method of claim 5, wherein the amount of water is at least five times the molar amount of said benzyl halide.

7. The method of one of claims 1 to 6, wherein said organic solvent is a water-miscible organic solvent.

8. The method of claim 7, wherein said organic solvent is methanol, ethanol, isopropanol, tetrahydrofuran, dimethylformamide, acetonitrile or a mixture thereof.
